(19) Europäisches Patentamt
European Patent Office
Office européen des brevets

(11) Veröffentlichungsnummer: **0 024 616**
**B1**

(12) # EUROPÄISCHE PATENTSCHRIFT

(45) Veröffentlichungstag der Patentschrift: **09.02.83**

(51) Int. Cl.³: **C 07 C 93/26,** A 61 K 31/215

(21) Anmeldenummer: **80104733.3**

(22) Anmeldetag: **11.08.80**

(54) **1-(3'-Acyloxyphenyl)-2-aminoethanole, deren Säureadditionssalze, diese enthaltende Arzneimittel, sowie Verfahren zu ihrer Herstellung.**

(30) Priorität: **14.08.79 DE 2933005**

(43) Veröffentlichungstag der Anmeldung: **11.03.81 Patentblatt 81/10**

(45) Bekanntmachung des Hinweises auf die Patenterteilung: **09.02.83 Patentblatt 83/6**

(84) Benannte Vertragsstaaten: **AT BE CH DE FR GB IT LI LU NL SE**

(56) Entgegenhaltungen:
**DE-A-2 341 876**
**DE-B-2 152 058**
**FR-A-2 299 860**
**FR-M-7 177**

(73) Patentinhaber: **Klinge Pharma GmbH, Leopoldstrasse 16, D-8000 München 40 (DE)**

(72) Erfinder: **Henschler, Dietrich, Dr., Frankenstrasse 53, D-8700 Würzburg (DE)**
Erfinder: **Wagner, Josef, Dr., Gladiolenstrasse 4, D-8022 Grünwald (DE)**
Erfinder: **Hampel, Hans, Dr., Harthausener Strasse 5, D-8011 Neukeferloh (DE)**
Erfinder: **Eisenburger, Rolf, Dr., Gertrud von Calker Strasse 19, D-8019 Moosach (DE)**
Erfinder: **Grill, Helmut, Dr., Zugspitzstrasse 148, D-8011 Vaterstetten (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 616 B1

BUNDESDRUCKEREI BERLIN

1-(3'-Acycloxyphenyl)-2-aminoethanole, deren Säureadditionssalze, diese enthaltende Arzneimittel, sowie Verfahren zu ihrer Herstellung

DL-1-(3'-Hydroxyphenyl)-2-aminoethanol, bekannt unter der Bezeichnung Norfenefrin (INN), stimuliert aufgrund seiner α-mimetischen Eigenschaften den Tonus kontraktiler Gefäßwandelelemente, was zur Erhöhung des Blutdruckes führt. Der Wirkstoff wird deshalb zur Behandlung von Erkrankungen aus dem hypotonen Formenkreis eingesetzt.

Von Nachteil ist seine geringe orale Wirksamkeit (E. Muscholl, Archiv. klin. exper. Ohren-, Nasen- u. Kehlkopfheilk. 188, 84–114 [Kongreßber., 1967]). Es hat daher nicht an Bemühungen gefehlt, durch Derivatisierung des Norfenefrins zu besser wirksamen Verbindungen zu kommen.

Bekanntgeworden sind Verbindungen vom Typ des N-Acyl-Norfenefrens, die gegebenenfalls am alkoholischen und auch am phenolischen Hydroxyl acyliert sein können (DE-PS 1 543 522, DE-OS 1 593 828, DE-OS 1 593 834). Allerdings zeigten diese Verbindungen im Tierversuch nach oraler Applikation keine pressorische Wirkung. Aus der D-PS 115 489 und der FR-PS 2 299 860 ist bekannt, daß man die an der Aminogruppe des Norfenefrins durch eine Methyl- oder Äthylgruppe substituierten Derivate (Phenylephrin und Etilefrin) an der phenolischen Hydroxylgruppe acylieren kann. Allerdings handelt es sich hierbei um Wirkstoffe, die aufgrund der Substitution an der Aminogruppe physiologisch anders wirken, so daß hieraus keine Rückschlüsse auf Norfenefrinderivate gezogen werden konnten.

Die der Erfindung zugrunde liegende Aufgabe bestand somit darin, solche pressorisch wirksamen Norfenefrinderivate herzustellen, die auch bei enteraler Anwendung eine gegenüber Norfenefrin überlegene α-mimetische Wirkung entfalten und bei erhöhter Lipophilität eine verbesserte Resorbierbarkeit besitzen.

Es wurde nun gefunden, daß Verbindungen der allgemeinen Formel I

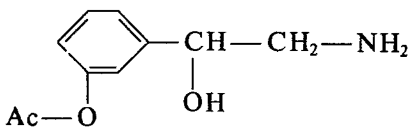

(I)

bei der Ac die in Anspruch 1 angegebene Bedeutung besitzt, bei intraduodenaler Applikation in ihrer Wirksamkeit sowohl dem Norfenefrin als auch seinen N-Acylderivaten überlegen sind. Wie noch gezeigt wird, läßt sich dies eindeutig am Blutdruck der Katze nachweisen. Im Gegensatz zu den bereits bekannten N-Acylderivaten des Norfenefrins, entfalten die erfindungsgemäßen Verbindungen eine pressorische Wirkung nicht nur nach intraduodenaler Applikation, sondern auch nach intravenöser Anwendung. Auf Grund ihrer gegenüber Norfenefrin verlängerten Wirkungsdauer, können sie in vorteilhafter Weise auch in Injektionsform angewendet werden.

Gegenstand der Erfindung sind 3'-O-Acylderivate des Norfenefrins, deren physiologisch verträgliche Säureadditionssalze, diese enthaltende Arzneimittel, sowie Verfahren zu ihrer Herstellung. Diese neuen Verbindungen sind äußerst wertvoll für die Behandlung von Erkrankungen aus dem hypotonen Formenkreis, vor allem der orthostatischen Dysregulation.

Die Herstellung der neuen Verbindungen erfolgt durch hydrogenolytische Entbenzylierung und Reduktion von Acetophenonderivaten der allgemeinen Formel II

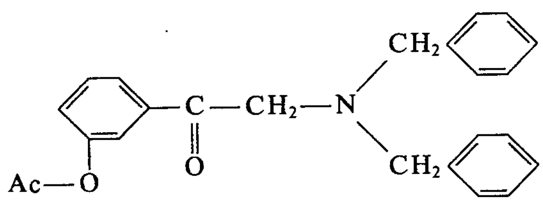

(II)

in der Ac die in Anspruch 1 gegebene Bedeutung besitzt. Für die Umsetzung der Acetophenonderivate nach Formel II zu den erfindungsgemäßen Verbindungen, eignet sich besonders die katalytische Hydrierungsreaktion in Gegenwart von Palladiumkohle. Zur Reduktion der Ketogruppe können jedoch auch komplexe Metallhydride, wie z. B. Natriumborhydrid eingesetzt werden.

Die für das Verfahren benötigten Ausgangsverbindungen der Formel II, können durch Acylierung der Verbindung nach Formel IV

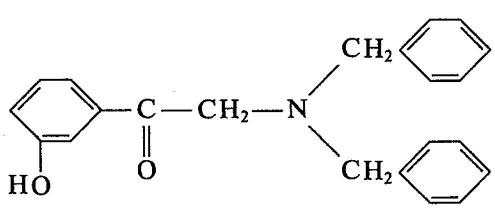

(IV)

mit einem Säurechlorid der allgemeinen Formel V

$$Ac-Cl \qquad (V)$$

wobei Ac die in Anspruch 1 genannte Bedeutung besitzt, erhalten werden.

Die erfindungsgemäßen Verbindungen der Formel I können auf übliche Weise in ihre physiologisch verträglichen Säureadditionssalze überführt werden. Geeignete Säuren sind beispielsweise Salzsäure, Bromwasserstoffsäure, Schwefelsäure, Methansulfonsäure, Essigsäure, Weinsäure, Zitronensäure, Benzoesäure od. dgl.

Die erfindungsgemäßen Verbindungen liegen in Form optisch aktiver Isomerer vor, die nach herkömmlichen Standardmethoden getrennt werden können.

Die Verbindungen der allgemeinen Formel I, sowie deren Säureadditionssalze können sowohl enteral als auch parenteral angewandt werden. Die Anwendung erfolgt bevorzugt in Dosen von 5—50 mg.

Die Wirkstoffe können in üblicher Form zur oralen Verabreichung konfektioniert werden, z. B. in Kapseln, in flüssiger Form, als Tabletten, Dragees oder als Pulver. Durch Vermischen mit festen, pulverförmigen Trägerstoffen wie Lactose, Saccharose, Sorbit, Mannit, Kartoffel- oder Maisstärke, Cellulosederivaten oder Gelatine, gegebenenfalls unter Zusatz von Gleitmitteln wie z. B. Magnesium- oder Calciumstearat oder Polyethylenglykolen, können sie zu Drageekernen oder Tabletten verarbeitet werden.

Als weitere Verabreichungsformen eignen sich Steckkapseln, z. B. aus Hartgelatine. Die Steckkapseln enthalten den Wirkstoff vorzugsweise als Granulat, z. B. in Mischung mit Füllstoffen wie Lactose, Saccharose, Mannit, Stärken, wie z. B. Kartoffelstärke, Maisstärke oder Amylopectin, Cellulosederivaten, oder mit Stoffen die zur Erzielung einer Depotwirkung angewendet werden.

Injektionsflüssigkeiten können mit Hilfe von physiologisch verträglichen Hilfsstoffen als Lösungen hergestellt werden.

Im folgenden wird die Erfindung anhand von Ausführungsbeispielen näher erläutert.

### Beispiel 1

1-(3′-Acetoxyphenyl)-2-aminoethanol-hydrochlorid

a) Herstellung des Ausgangsproduktes

3′-Acetoxy-ω-dibenzylamino-acetophenon-hydrochlorid

Zu einer Suspension von 55,2 g (0,15 Mol) 3′-Hydroxy-ω-dibenzylamino-acetophenon-hydrochlorid in 150 ml Eisessig werden unter Rühren bei 60°C innerhalb 0,5 h 53 ml (0,75 Mol) Acetylchlorid zugetropft und bei gleicher Temperatur noch 1 h gerührt. Anschließend wird das Reaktionsgemisch zur Trockne eingeengt und der ölige Rückstand in ein Gemisch aus 230 ml konz. Ammoniak, 1 kg Eis und 600 ml Ether eingerührt. Die etherische Phase wird mit kaltem Wasser neutral gewaschen und über Natriumsulfat getrocknet. Nach dem Verdampfen des Ethers wird der gelbe, ölige Rückstand in 600 ml Methanol gelöst und mit konz. Salzsäure auf pH 2 eingestellt. Die saure Lösung wird auf ca. 300 ml eingeengt und das Produkt mit 1,5 l Ether zur Fällung gebracht. Farblose Kristalle.

Ausbeute: 57,5 g (93,9%)
$R_f$ 0,8 [Chloroform/Methanol (9 : 1)]

b) Erfindungsgemäße Herstellung von 1-(3′-Acetoxyphenyl)-2-aminoethanol-hydrochlorid

42 g (0,1 Mol) 3′-Acetoxy-ω-dibenzylamino-acetophenonhydrochlorid werden in 600 ml Methanol bei Raumtemperatur und Normaldruck in Gegenwart von 12,6 g feuchter, 10proz. Palladiumkohle ca. 2,5 h bis zum Stillstand der Wasserstoffaufnahme hydriert. Anschließend wird der Katalysator abgetrennt und das Lösungsmittel abdestilliert. Der Rückstand wird zunächst aus Methanol/Ether umgefällt und anschließend aus Isopropanol kristallisiert.

Farblose Kristalle vom Schmp. 119—121°C.
Ausbeute: 17,7 g (76%)
$R_f$ 0,3 [Chloroform/Methanol/Wasser (59 : 33 : 8)]

$C_{10}H_{14}ClNO_3$ (231,1)

| | | | |
|---|---|---|---|
| Ber. | C 51,83 | H 6,09 | N 6,05 |
| Gef. | C 51,77 | H 6,02 | N 6,05 |

Mol.-Gew. der freien Base: 195 (massenspektrometrisch bestimmt)[1])

[1]) Die Aufnahme der Massenspektren erfolgte mit dem Massenspektrometer Varian MAT 112 S mit Elektronenstoß-Ionisation.

Beispiel 2

1-(3'-Pivaloyloxyphenyl)-2-aminoethanol-hydrochlorid

Eine auf −5°C abgekühlte Lösung von 27,2 g (0,1 Mol) 3'-Pivaloyloxy-ω-amino-acetophenon-hydrochlorid in 300 ml Methanol wird unter Rühren vorsichtig mit 8,4 g (0,22 Mol) Natriumborhydrid versetzt, so daß die Reaktionstemperatur nicht über 0°C ansteigt. Das Reaktionsgemisch wird bei 0°C noch weitere 20 min gerührt und anschließend vorsichtig mit 60 ml Eisessig zersetzt. Nach dem Abdestillieren des Lösungsmittels wird der Rückstand mit 450 ml Wasser aufgenommen und unter Eiskühlung mit 60 ml konz. Ammoniak versetzt. Die freigesetzte Base wird in 450 ml Essigsäureethylester aufgenommen und mit kaltem Wasser neutral gewaschen. Die Lösung wird zur Trockne eingeengt, der farblos ölige Rückstand in Methanol aufgenommen und mit etherischer Salzsäure gefällt.

Farblose Kristalle vom Schmp. 144—146°C (Methanol/Ether)
Ausbeute: 17,7 g (65%)
$R_f$ 0,3 [Chloroform/Methanol/Wasser (59 : 33 : 8)]

$C_{13}H_{20}ClNO_3$ (273,8)

| | | | |
|---|---|---|---|
| Ber. | C 57,02 | H 7,36 | N 5,12 |
| Gef. | C 56,89 | H 7,22 | N 4,99 |

Mol.-Gew. der freien Base: 237 (massenspektrometrisch bestimmt)

Beispiel 3

1-(3'-Heptanoyloxyphenyl)-2-aminoethanol-hydrochlorid

a) Herstellung des Ausgangsproduktes

3'-Heptanoyloxy-ω-dibenzylamino-acetophenon-hydrochlorid

Zu einer Suspension von 55,2 g (0,15 Mol) 3'-Hydroxy-ω-dibenzylamino-acetophenon-hydrochlorid in 49,3 ml Trifluoressigsäure werden unter Rühren bei 65°C innerhalb 1 h 69,7 ml (0,45 Mol) Heptanoylchlorid zugetropft und bei gleicher Temperatur noch 1 h gerührt. Nach dem Abkühlen auf Raumtemperatur wird das Reaktionsgemisch auf 1 kg Eis gegossen, dann mit 250 ml konz. Ammoniak versetzt und schließlich mit 1 l Essigsäureethylester ausgeschüttelt. Die organische Phase wird mit kaltem Wasser neutral gewaschen und über Natriumsulfat getrocknet. Nach dem Verdampfen des Lösungsmittels wird der ölige Rückstand in 400 ml Methanol gelöst und mit konz. Salzsäure auf pH 2 eingestellt. Die saure Lösung wird auf 300 ml eingeengt und das Produkt mit 1,7 l Ether zur Fällung gebracht. Farbloses, amorphes Pulver.

Ausbeute: 63,4 g (88%)
$R_f$ 0,8 [Chloroform/Methanol (9 : 1)]

b) Erfindungsgemäße Herstellung von
1-(3'-Heptanoyloxyphenyl)-2-aminoethanol-hydrochlorid

48 g (0,1 Mol) 3'-Heptanoyloxy-ω-dibenzylamino-acetophenon-hydrochlorid werden in 800 ml Methanol bei Raumtemperatur und Normaldruck in Gegenwart von 20 g feuchter, 10proz. Palladiumkohle ca. 2,5 h, bis zum Stillstand der Wasserstoffaufnahme, hydriert. Anschließend wird der Katalysator abgetrennt und das Lösungsmittel abdestilliert. Der Rückstand wird zunächst aus Methanol/Ether umgefällt und schließlich aus Isopropanol kristallisiert.

Blaßgelbe Kristalle vom Schmp. 100—101°C
Ausbeute: 14,6 g (43,8%)
$R_f$ 0,3 [Chloroform/Methanol/Wasser (59 : 33 : 8)]

$C_{15}H_{24}ClNO_3$ (301,8)

| | | | |
|---|---|---|---|
| Ber. | C 59,69 | H 8,02 | N 4,64 |
| Gef. | C 59,80 | H 8,02 | N 4,58 |

Mol.-Gew. der freien Base: 265 (massenspektrometrisch bestimmt)

Beispiel 4

1-[3'-(4'-Methylbenzoyloxy)phenyl]-2-aminoethanol-hydrochlorid

a) Herstellung des Ausgangsproduktes

3'-(4'-Methylbenzoyloxy)-ω-dibenzylamino-acetophenonhydrochlorid

Zu einer Suspension von 55,2 g (0,15 Mol) 3'-Hydroxy-ω-dibenzylamino-acetophenon-hydrochlorid in 49,3 ml Trifluoressigsäure werden unter Rühren bei 75°C innerhalb 1 h 69,6 g (0,45 Mol) 4-Methylbenzoesäurechlorid zugetropft und bei gleicher Temperatur noch 1 h gerührt. Nach dem Abkühlen auf Raumtemperatur wird das Reaktionsgemisch auf 1 kg Eis gegossen, dann mit 200 ml konz. Ammoniak versetzt und schließlich mit 1 l Essigsäureethylester ausgeschüttelt. Die organische Phase wird mit kaltem Wasser neutral gewaschen und über Natriumsulfat getrocknet. Nach dem Verdampfen des Lösungsmittels wird der feste Rückstand in 500 ml Methanol suspendiert und mit konz. Salzsäure auf pH 2 eingestellt. Die saure Lösung wird auf ca. 300 ml eingeengt und das Produkt mit 1,5 l Ether zur Fällung gebracht. Farbloses, amorphes Pulver. Schmp. 140° C.

Ausbeute: 59,8 g (82%)
$R_f$ 0,8 [Chloroform/Methanol (9 : 1)]

b) Erfindungsgemäße Herstellung von
1-[3'-(4'-Methylbenzoyloxy)phenyl]-2-aminoethanol-hydrochlorid

48,6 g (0,1 Mol) 3'-(4'-Methylbenzoyloxy)-ω-dibenzylamino-acetophenon-hydrochlorid werden in 1,5 l Methanol bei 40° C und Normaldruck in Gegenwart von 15 g feuchter, 10proz. Palladiumkohle ca. 3 h, bis zum Stillstand der Wasserstoffaufnahme, hydriert. Anschließend wird der Katalysator abgetrennt und das Lösungsmittel abdestilliert. Der Rückstand wird aus Methanol/Ether umgefällt.

Farblose Kristalle vom Schmp. 165—167° C
Ausbeute: 23,6 g (76,6%)
$R_f$ 0,3 [Chloroform/Methanol/Wasser (59 : 33 : 8)]
$C_{16}H_{18}ClNO_3$ (307,8)

| | | | |
|---|---|---|---|
| Ber. | C 62,43 | H 5,89 | N 4,55 |
| Gef. | C 62,37 | H 5,82 | N 4,48 |

Mol.-Gew. der freien Base: 271 (massenspektrometrisch bestimmt)

In analoger Weise zu den Beispielen 1—4 wurden weitere 1-(3'-Acyloxyphenyl)-2-aminoethanole der allgemeinen Formel I hergestellt und in nachfolgender Tabelle mit physikalischen Kenndaten aufgeführt. Aus Gründen der Vollständigkeit werden in der Tabelle die in den Beispielen beschriebenen Verbindungen nochmals angegeben.

In Tab. 2 werden nachsynthetisierte N-Acylderivate des Norfenefrins aufgeführt, die von den deutschen Anmeldungen 1 543 522, 1 593 828 und 1 593 834 beansprucht werden.

Tabelle 1

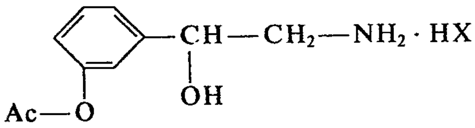

| No. | Ac | $X^-$ | Schmp. (°C) | Mindest-löslichkeit in Wasser (%) |
|---|---|---|---|---|
| 1 | $CH_3CO$ | Cl | 119 bis 121 (a) | 50 |
| 2 | $CH_3CH_2CO$ | Cl | 96 bis 99 (b) | 50 |
| 3 | $CH_3(CH_2)_2CO$ | Cl | 100 bis 101 (b) | 50 |
| 4 | $(CH_3)_2CHCO$ | Cl | 92 bis 94 (b) | 50 |
| 5 | $CH_3(CH_2)_3CO$ | Cl | 98 bis 99 (b) | 50 |
| 6 | $(CH_3)_2CHCH_2CO$ | Cl | 100 bis 101 (b) | 50 |
| 7 | $(CH_3)_3CCO$ | Cl | 144 bis 146 (b) | 50 |
| 8 | $CH_3(CH_2)_4CO$ | Cl | 103 bis 105 (b) | 50 |
| 9 | $(CH_3)_2CHCH_2CH_2CO$ | Cl | 96 bis 99 (b) | 50 |
| 10 | $(CH_3CH_2)_2CHCO$ | $CH_3SO_3$ | 120 bis 121 (b) | 50 |
| 11 | $(CH_3)_3CCH_2CO$ | Cl | 130 bis 135 (b) | 50 |
| 12 | $CH_3(CH_2)_5CO$ | Cl | 100 bis 101 (a) | 50 |
| 13 | $CH_3CH_2CH_2C(CH_3CH_2)_2CO$ | $CH_3SO_3$ | 122 bis 123 (b) | 50 |
| 14 | $CH_3(CH_2)_{10}CO$ | Cl | 107 bis 109 (a) | 15 |
| 15 | $C_6H_5CH_2CO$ | Cl | 145 bis 149 (b) | 50 |
| 16 | $C_6H_5(CH_3)CHCO$ | $CH_3SO_3$ | 135 bis 137 (b) | 50 |
| 17 | $C_6H_5(CH_3CH_2)CHCO$ | Cl | 94 bis 97 (b) | 20 |
| 18 | $C_6H_5CO$ | Cl | 128 bis 132 (b) | 50 |
| 19 | 2-$CH_3C_6H_4CO$ | Cl | 92 bis 94 (b) | 50 |
| 20 | 4-$CH_3C_6H_4CO$ | Cl | 165 bis 167 (b) | 50 |

Kristalle aus a = Isopropanol.
b = Methanol/Ether.

Tab. 2

Bekannte N-Acylderivate des Norfenefrins

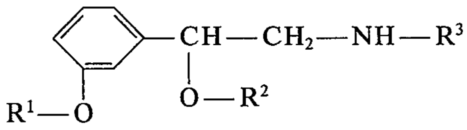

| No. | $R^1$ | $R^2$ | $R^3$ | Schmp. (°C) | Mindestlöslichkeit in Wasser (%) |
|---|---|---|---|---|---|
| 21 | H | H | $CH_3CO$ | 115 bis 116 (a)<br>118 bis 119 (DE-PS 15 43 522) | 10 |
| 22 | H | $CH_3CO$ | $CH_3CO$ | 107 bis 109 (c)<br>107 bis 108 (DE-OS 15 93 834) | 1.2 |
| 23 | H | H | $(CH_3)_3CCO$ | 120 bis 122 (c)<br>115 bis 117 (DE-OS 15 43 522) | 1 |
| 24 | H | $(CH_3)_3CCO$ | $(CH_3)_3CCO$ | 155 bis 157 (c) | 0.01 |
| 25 | $(CH_3)_3CCO$ | $(CH_3)_3CCO$ | $(CH_3)_3CCO$ | 84 bis 86 (d) | 0.01 |

Kristalle aus a = Isopropanol.
c = Essigester.
d = Petrolether.

Beispiel 8

Pharmakologische Testung

1. Blutdrucksteigernde Wirkung nach intraduodenaler Applikation

An 2–4 kg schweren, männlichen und weiblichen, narkotisierten Katzen [Chloralose (70 mg/kg) und Urethan 25proz. (200 mg/kg) i. p.] wurden die Substanzen in wäßriger Lösung intraduodenal appliziert. Der Anstieg des systolischen und des diastolischen Blutdrucks wird in Prozenten des Ausgangswertes angegeben. N bedeutet Anzahl der Versuchstiere pro Versuch.

Tab. 3

| Verbindung | N | Dosis mg/kg | Systol. Blutdruck | Diastol. Blutdruck |
|---|---|---|---|---|
| Norfenefrin | 8 | 3 | 25 ± 9 | 18 ± 6 |
| | 8 | 10 | 34 ± 7 | 25 ± 5 |
| | 6 | 30 | 41 ± 7 | 37 ± 9 |
| 1 | 6 | 1 | 27 ± 10 | 20 ± 10 |
| | 6 | 3 | 35 ± 8 | 29 ± 6 |
| | 6 | 10 | 59 ± 6 | 48 ± 8 |
| 5 | 5 | 0.3 | 20 ± 10 | 19 ± 11 |
| | 6 | 1 | 33 ± 7 | 31 ± 8 |
| | 6 | 3 | 42 ± 10 | 29 ± 8 |
| 7 | 7 | 1 | 17 ± 7 | 17 ± 6 |
| | 7 | 3 | 37 ± 6 | 29 ± 5 |
| | 7 | 10 | 79 ± 8 | 74 ± 13 |
| 10 | 6 | 1 | 25 ± 5 | 19 ± 3 |
| | 6 | 3 | 48 ± 11 | 48 ± 11 |
| | 6 | 10 | 85 ± 11 | 89 ± 14 |
| 12 | 6 | 1 | 21 ± 8 | 17 ± 7 |
| | 6 | 3 | 70 ± 14 | 65 ± 17 |
| | 6 | 10 | 61 ± 10 | 51 ± 11 |
| 14 | 7 | 1 | 23 ± 6 | 16 ± 4 |
| | 7 | 3 | 39 ± 9 | 25 ± 8 |
| | 6 | 10 | 87 ± 15 | 90 ± 25 |
| 15 | 7 | 1 | 24 ± 3 | 20 ± 4 |
| | 6 | 3 | 50 ± 11 | 36 ± 11 |
| | 5 | 10 | 88 ± 15 | 81 ± 15 |
| 16 | 6 | 1 | 20 ± 7 | 10 ± 3 |
| | 6 | 3 | 38 ± 12 | 31 ± 8 |
| | 6 | 10 | 63 ± 9 | 55 ± 13 |
| 17 | 7 | 1 | 29 ± 7 | 25 ± 7 |
| | 6 | 3 | 42 ± 9 | 31 ± 6 |
| | 6 | 10 | 47 ± 10 | 38 ± 6 |
| 19 | 6 | 1 | 17 ± 5 | 15 ± 7 |
| | 7 | 3 | 26 ± 7 | 21 ± 6 |
| | 7 | 10 | 37 ± 7 | 32 ± 11 |
| 20 | 6 | 0.3 | 20 ± 10 | 17 ± 10 |
| | 6 | 1 | 28 ± 6 | 26 ± 6 |
| | 7 | 3 | 122 ± 25 | 100 ± 24 |
| N-Acylderivate | | | | |
| 21 | 6 | 30 | 7 ± 3 | 5 ± 2 |
| 23 | 6 | 30 | 7 ± 3 | 7 ± 3 |
| 24 | 6 | 30 | 0 | 0 |
| 25 | 6 | 30 | 0 | 0 |

2. Blutdrucksteigernde Wirkung nach intravenöser Applikation

An 2–4 kg schweren, männlichen und weiblichen, narkotisierten Katzen [Chloralose (70 mg/kg) und Urethan 25proz. (200 mg/kg) i. p.] wurden die Substanzen in wäßriger Lösung intravenös appliziert. Der Anstieg des systolischen und des diastolischen Blutdruckes wird in Prozenten des Ausgangswertes angegeben. N bedeutet Anzahl der Versuchstiere pro Versuch. Die Wirkungsdauer ist in Minuten angegeben.

Tab. 4

| Verbindung | N | Dosis mg/kg | Systol. Blutdruck | Diastol. Blutdruck | Wirkungs-dauer |
|---|---|---|---|---|---|
| Norfenefrin | 6 | 0.01 | 9 ± 3 | 5 ± 3 | 9 ± 2 |
| | 6 | 0.03 | 44 ± 9 | 39 ± 7 | 19 ± 9 |
| | 6 | 0.10 | 88 ± 20 | 78 ± 21 | 24 ± 5 |
| 1 | 6 | 0.10 | 57 ± 13 | 48 ± 12 | 28 ± 4 |
| 7 | 6 | 0.10 | 72 ± 21 | 60 ± 16 | 35 ± 7 |
| 12 | 6 | 0.10 | 54 ± 7 | 41 ± 7 | 30 ± 6 |
| 14 | 6 | 0.10 | 37 ± 10 | 32 ± 10 | 33 ± 7 |
| 19 | 6 | 0.10 | 36 ± 6 | 36 ± 6 | 27 ± 7 |
| 20 | 8 | 0.10 | 21 ± 6 | 15 ± 5 | 27 ± 8 |
| N-Acylderivate | | | | | |
| 21 | 6 | 0.30 | 0 | 0 | 0 |
| | 6 | 1.00 | 0 | 0 | 0 |
| 23 | 6 | 0.30 | 0 | 0 | 0 |
| | 6 | 1.00 | 0 | 0 | 0 |

**Patentansprüche für die Vertragsstaaten BE, CH, LI, DE, FR, GB, IT, LU, NL, SE**

1. 1-(3'-Acyloxyphenyl)-2-aminoethanole der allgemeinen Formel I

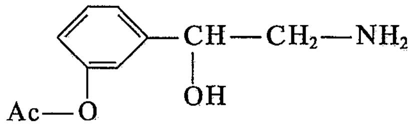

(I)

in der Ac den Acylrest einer geradkettigen oder verzweigten Fettsäure mit bis zu 12 Kohlenstoffatomen darstellt, ein Benzoylrest sein kann, der gegebenenfalls eine Methylgruppe trägt, oder ein α-Phenylalkanoylrest ist, dessen Alkylgruppe bis zu drei Kohlenstoffatome enthalten kann, und deren physiologisch verträgliche Säureadditionssalze.

2. Verfahren zur Herstellung von Verbindungen nach Anspruch 1, dadurch gekennzeichnet, daß man in an sich bekannter Weise

a) eine Verbindung der allgemeinen Formel II

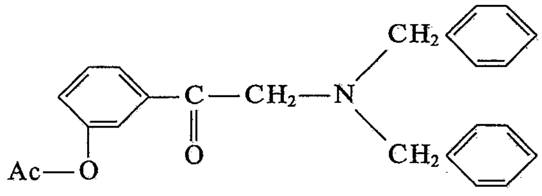

(II)

in der Ac die in Anspruch 1 gegebene Bedeutung besitzt, in einem Lösungsmittel unter Verwendung von Palladiumkohle hydrogenolytisch entbenzyliert und zum Alkohol reduziert, oder

b) eine Verbindung der allgemeinen Formel III

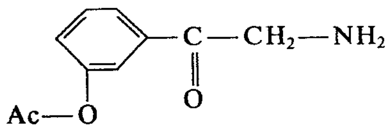

(III)

in der Ac die in Anspruch 1 gegebene Bedeutung besitzt, in einem Lösungsmittel unter Verwendung von Natriumborhydrid zum Alkohol reduziert.

3. Arzneimittel, enthaltend eine Verbindung gemäß Anspruch 1.

**Patentanspruch für den Vertragsstaat AT**

Verfahren zur Herstellung von 1-(3'-Acyloxyphenyl)-2-minoethanole der allgemeinen Formel I

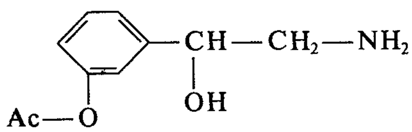

(I)

in der Ac den Acylrest einer geradkettigen oder verzweigten Fettsäure mit bis zu 12 Kohlenstoffatomen darstellt, ein Benzoylrest sein kann, der gegebenenfalls eine Methylgruppe trägt, oder ein $\alpha$-Phenylalkanoylrest ist, dessen Alkylgruppe bis zu drei Kohlenstoffatome enthalten kann, und deren physiologisch verträgliche Säureadditionssalze, dadurch gekennzeichnet, daß man in an sich bekannter Weise

a) eine Verbindung der allgemeinen Formel II

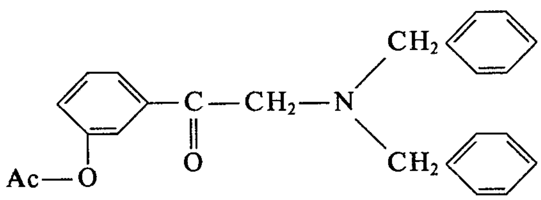

(II)

in der Ac die im Oberbegriff gegebene Bedeutung besitzt, in einem Lösungsmittel unter Verwendung von Palladiumkohle hydrogenolytisch entbenzyliert und zum Alkohol reduziert, oder

b) eine Verbindung der allgemeinen Formel III

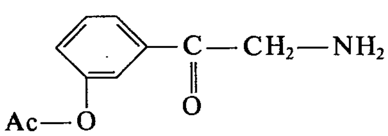

(III)

in der Ac die im Oberbegriff gegebene Bedeutung besitzt, in einem Lösungsmittel unter Verwendung von Natriumborhydrid zum Alkohol reduziert.

**Claims for the contracting states: BE, CH, LI, DE, FR, GB, IT, LU, NL, SE**

1. 1-(3'-acyloxyphenyl)-2-aminoethanols represented by the general formula I

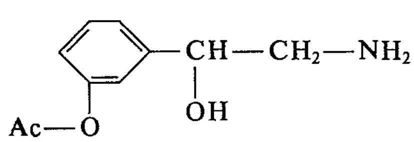

(I)

in which Ac is the acyl radical of a straight chain or a branched fatty acid with up to 12 carbon atoms, or a benzoyl radical optionally having a methyl group, or an alpha-phenylalkanoyl radical whose alkyl group can have up to three carbon atoms, and the physiologically acceptable acid addition salts thereof.

2. A method of preparing compounds according to claim 1 characterized in that, in a manner known per se,

a) a compound represented by the general formula II

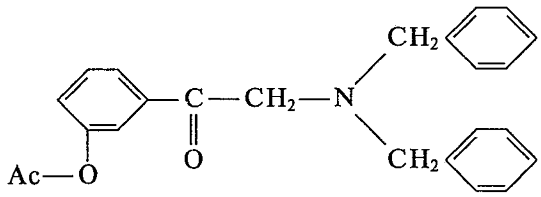

(II)

in which Ac has the meaning indicated in claim 1, is hydrogenolytically de-benzylated in a solvent with the use of palladium-charcoal and reduced to the alcohol, or

b) a compound represented by the general formula III

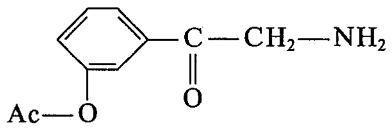

(III)

in which Ac has the meaning indicated in claim 1, is reduced to the alcohol in a solvent with the use of sodium borohydride.

3. A pharmaceutical preparation containing a compound as claimed in claim 1.

**Claim for the contracting state: AT**

A process for the preparation of 1-(3′-acyloxyphenyl)-2-aminoethanols represented by the general formula I

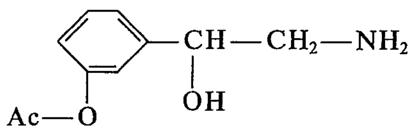

(I)

in which Ac is the acyl radical of a straight chain or a branched fatty acid with up to 12 carbon atoms, or a benzoyl radical optionally having a methyl group, or an alpha-phenylalkanoyl radical whose alkyl group can have up to three carbon atoms, and the physiologically acceptable acid addition salts thereof characterized in that, in a manner known per se

a) a compound represented by the general formula II

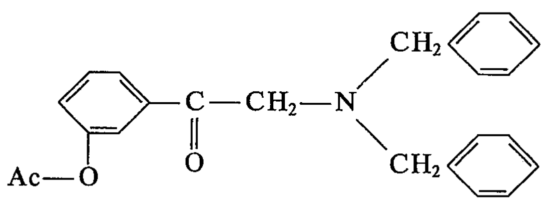

(II)

in which Ac has the meaning indicated in claim 1, is hydrogenolytically de-benzylated in a solvent with the use of palladium charcoal and reduced to the alcohol, or

b) a compound represented by the general formula III

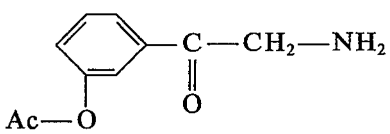

(III)

in which Ac has the meaning indicated in claim 1, is reduced to the alcohol in a solvent with the use of sodium borohydride.

**Revendications pour les etats contractants: BE, CH, LI, DE, FR, GB, IT, LU, NL, SE**

1. 1-(3'-Acyloxyphényl)-2-aminoéthanols de formule générale

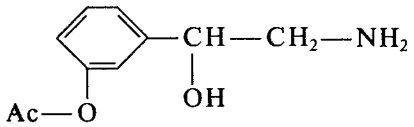

(I)

où Ac représente le radical acyle d'un acide gras à chaîne droite ou ramifiée ayant jusqu'à 12 atomes de carbone, peut être un radical benzoyle portant éventuellement un groupe méthyle, ou un radical α-phénylalcanoyle dont le groupe alcoyle peut contenir jusqu'à 3 atomes de carbone, et leurs sels d'addition d'acides physiologiquement acceptables.

2. Procédé de préparation de composés selon la revendication 1 caractérisé en ce que, de façon classique,

a) on débenzyle par hydrogénolyse et on réduit en alcool un composé de formule générale II

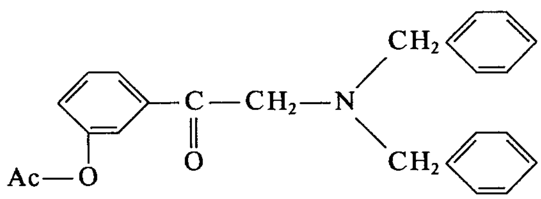

(II)

où Ac a la signification donnée dans la revendication 1, dans un solvant en utilisant du palladium-charbon, ou

b) on réduit en alcool un composé de formule générale III

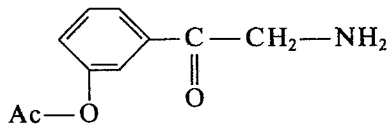

(III)

où Ac a la signification donnée dans la revendication 1, dans un solvant en utilisant du borohydrure de sodium.

3. Médicament contenant un composé selon la revendication 1.

**Revendication pour l'etat contractant: AT**

Procédé de préparation de 1-(3'-acyloxyphényl)-2-aminoéthanols de formule générale I

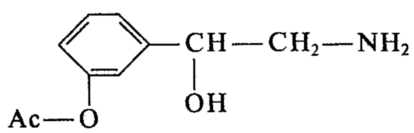

(I)

où Ac représente le radical acyle d'un acide gras à chaîne droite ou ramifiée ayant jusqu'à 12 atomes de carbone, peut être un benzyle, qui porte éventuellement un groupe méthyle, ou un radical α-phénylalcanoyle dont le groupe alcoyle peut contenir jusqu'à 3 atomes de carbone, et de leurs sels d'addition d'acides physiologiquement acceptables, caractérisé en ce que de façon classique

a) on débenzyle par hydrogénolyse et on réduit en alcool un composé de formule générale II

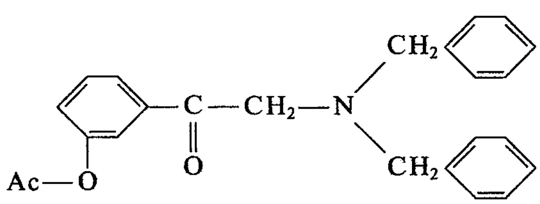

(II)

où Ac a la signification donnée dans le préambule, dans un solvant en utilisant du palladium-charbon, ou

b) on réduit en alcool un composé de formule générale III

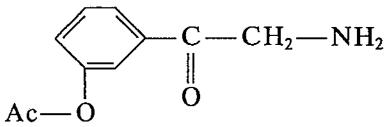

(III)

où Ac a la signification donnée dans le préambule, dans un solvant en utilisant du borohydrure de sodium.